# EUROPÄISCHE PATENTSCHRIFT

(11) **EP 1 761 777 B1**
(45) Veröffentlichungstag und Bekanntmachung des Hinweises auf die Patenterteilung: **16.11.2011**
(21) Anmeldenummer: 05756668.9
(22) Anmeldetag: 23.06.2005
(51) Int. Cl.: G01N 33/569

(54) **SEPSISDIAGNOSETEST**
SEPSIS DIAGNOSTIC TEST
TEST DE DIAGNOSTIC DE LA SEPTICEMIE

(30) Priorität: 29.06.2004 DE 102004031560
(43) Veröffentlichungstag der Anmeldung: 14.03.2007
(73) Patentinhaber: Universität Rostock, 18051 Rostock (DE)
(72) Erfinder: MITZNER, Steffen, 18119 Rostock (DE); ALTRICHTER, Jens, 18196 Kavelstorf (DE); DOLLMANTEL, Hans-Joachim, 18059 Rostock (DE); SULZ, Jana, Dr., 18069 Rostock (DE)
(74) Vertreter: WSL Patentanwälte
(86) Internationale Anmeldenummer: PCT/EP2005/052950
(87) Internationale Veröffentlichungsnummer: WO 2006/000570

(56) Entgegenhaltungen:
- PASCUAL C ET AL: "A controlled study of leukocyte activation in septic patients." INTENSIVE CARE MEDICINE. JUL 1997, Bd. 23, Nr. 7, Juli 1997 (1997-07), Seiten 743-748, XP002341157 ISSN: 0342-4642
- MITZNER S R ET AL: "Use of human preconditioned phagocytes for extracorporeal immune support: introduction of a concept." THERAPEUTIC APHERESIS : OFFICIAL JOURNAL OF THE INTERNATIONAL SOCIETY FOR APHERESIS AND THE JAPANESE SOCIETY FOR APHERESIS. OCT 2001, Bd. 5, Nr. 5, Oktober 2001 (2001-10), Seiten 423-432, XP002341156 ISSN: 1091-6660
- ARMSTRONG D. ET AL: "The analysis of free radicals, lipid peroxides, antioxidant enzymes and compounds related to oxidative stress as applied to the clinical chemistry laboratory", ADVANCES IN EXPERIMENTAL MEDICINE AND BIOLOGY, vol. 366, 1994, pages 43-58,

## Beschreibung

### Gegenstand der Erfindung

Die Erfindung betrifft ein Verfahren zur extrakorporalen qualitativen oder semiquantiativen Bestimmung der Menge an Indikatoren für SIRS bzw. Sepsis im Blut, Blutserum, Blutplasma, anderen Körperflüssigkeiten oder Lavagen oder Bestandteilen davon von menschlichen oder tierischen Subjekten. Des weiteren betrifft die Erfindung ein entsprechendes Test-Kit, welches die zur Durchführung des erfindungsgemäßen Verfahrens erforderlichen Bestandteile enthält.

### Hintergrund der Erfindung

Die Pathogenese von SIRS (Systemic Inflammatory Response System) und Sepsis bis zu den vorletalen Stadien des septischen Schocks und multiplen Organversagens wird im wesentlichen auf eine Dysfunktion des Immunsystems zurückgeführt (Grimminger F et al. 1997). Eine zuverlässige Verlaufsprognose und erfolgreiche immunmodulatorische Therapie von SIRS/Sepsis setzen eine gut definierte Stadieneinteilung und dafür geeignete diagnostische Methoden voraus.

Nur in groben Umrissen läßt sich bis jetzt eine hyperinflammatorische von einer antiinflammatorischen Phase anhand der Verlaufskinetik von einigen Cytokinen unterscheiden (Grimminger F et al. 1997, Romaschin AD et al. 1998). Aufgrund der hohen Variabilität im Cytokinspiegel der Patienten und des komplexen Beziehungsgeflechtes zwischen den einzelnen Cytokinen hat sich auf diesem Gebiet bis jetzt kein praktischer Nutzen für Diagnostik und Therapie ergeben. Andere Inflammations-Marker wie CRP und Procalcitonin (Oberhoffer M et al. 1999, Mimoz O et al. 1998, Al-Nawas B et al. 1995/96) haben zwar einen relativ hohen Prädiktionswert für den Ausgang des Krankheitsverlaufs, geben aber keinerlei Indikationen für eine immunmodulatorische Behandlung.

Auf der zellulären Ebene des Immunsystems wird den Monozyten und (neutrophilen) Granulozyten eine wichtige Rolle beim SIRS/Sepsis-Verlauf beigemessen (Nussler AK et al. 1999). Monozyten und ihnen verwandte Zellen (z.B. Makrophagen, dentritische Zellen) initiieren die Immunabwehr durch Antigenpräsentation und Auschüttung von Lymphozyten stimulierenden Cytokinen. Das Ausmaß der HLA-DR-Expression auf zirkulierenden Monozyten wird als Parameter zur Prognose des Sepsis-Risikos bei Trauma- und SIRS-Patienten und der Schwere des Krankheitsverlaufs bei eingetretener Sepsis (Letalitätsrisiko) genutzt (Wakefield CH et al. 1993, Asadullahn K et al. 1995). Diese HLA-DR-Diagnostik läßt sich zudem mit einer immunmodulatorischen Therapie koppeln. Mit einer Verabreichung von spezifischen Cytokinen (IFN-G, G-CSF), deren Dosierung sich nach der Steigerung der HLA-DR-Expression richtet, kann ein fataler Krankheitsverlauf verhindert werden (Döcke WD et al. 1997).

Darüber hinaus sind aktivierte Monozyten (Makrophagen) gemeinsam mit den (neutrophilen) Granulozyten zur unmittelbaren Elimination von Erregern durch Phagozytose (Fresszellen) und die Ausschüttung von reaktiven Sauerstoffintermediaten (ROI = Reactive Oxygen Intermediates) und Stickoxiden befähigt. Aufgrund chemotaktischer Reize können beide Zelltypen die Zirkulation verlassen und nach Gewebsinfiltration ihre mikrobizide Aktivität entfalten.

Diese elementaren Abwehrfunktionen der Zellen sind sowohl von endogenen Faktoren, wie z. B. dem Reifungszustand der Zellen, und in noch höherem Ausmaß von exogenen Faktoren abhängig, wie Cytokinen, Chemokinen, Metaboliten, Endotoxinen und anderen Erregerprodukten. Aufgrund dieser komplexen Beziehungen steht die Untersuchung dieser Leukozytenfunktionen während des SIRS/Sepsis-Verlaufs vor erheblichen methodischen Schwierigkeiten.

Phagozytose-Aktivität und ROI-Produktion (Oxyburst) werden in der Regel nur bei peripheren Blutleukozyten bestimmt, da die diagnostisch interessanteren residenten Gewebsleukozyten für diese Bestimmungen mit wenigen Ausnahmen (z. B. Peritoneal-Makrophagen) kaum zugänglich sind (Dong YL et al. 1993, Holzer K et al. 2000). Zudem werden Phagozytose und Oxyburst erst durch den Zusatz von "Standard"-Stimulantien ausgelöst (Bakterien oder Mikropartikel, f-MLP, PMA) (EP 0 435 226, DE 41 17 459), deren Wirkung spezifisch krankheitsbedingte Modulatoren der Leukozytenaktivität im Patientenplasma überdecken kann.

Der umgekehrte Weg, den Einfluß von Patientenplasma und Faktoren daraus auf die Aktivität von gesunden Kontroll-Leukozyten zu untersuchen, scheitert an der veränderten Charakteristik isolierter, von autologem Plasma getrennter Leukozyten (Pascual C et al. 1997) und der schwierigen Standardisierung aufgrund der Heterogenität derartiger Populationen.

### Aufgabe der Erfindung

Die Aufgabe der Erfindung bestand darin, einen gegenüber dem Stand der Technik verbesserten Test bereitzustellen, mit dem in einer Probe, wie z. B. Blutserum eines Patienten, das Vorliegen und/oder die Schwere von SIRS oder Sepsis schnell, preiswert, zuverlässig und reproduzierbar festgestellt werden kann.

### Beschreibung der Erfindung

Die erfindungsgemäße Aufgabe wird gelöst durch ein Verfahren zur extrakorporalen qualitativen oder semiquantiativen Bestimmung der Menge an Indikatoren für Systemic Inflammatory Response System (SIRS) bzw. Sepsis im Blut, Blutserum, Blutplasma, anderen Körperflüssigkeiten oder Lavagen oder Bestandteilen davon von menschlichen oder tierischen Subjekten, wobei das Verfahren die Stufen umfaßt, in denen man
- eine permanente, differenzierte leukozytäre Zellinie in Kultur bereitstellt, die ohne zusätzliche auslösende Agenzien auf Indikatoren für das Vorhandensein oder Fehlen von Indikatoren für SIRS bzw. Sepsis in einer Probe unter Freisetzung von Sauerstoffintermediaten (ROI) anspricht,
- in wenigstens einem ersten Ansatz Zellen der Zellinie mit Blut, Blutserum oder Blutplasma, anderen Körperflüssigkeiten oder Lavagen oder Bestandteilen davon eines zu untersuchenden menschlichen oder tierischen Subjektes und einem Reagenz, welches auf Sauerstoffintermediate (ROI) anspricht und eine Farb-, Licht- oder eine andere meßbare Reaktion eingeht, in Kontakt bringt,
- in wenigstens einem weiteren Ansatz Zellen der Zellinie mit Blut, Blutserum oder Blutplasma, anderen Körperflüssigkeiten oder Lavagen oder Bestandteilen davon eines menschlichen oder tierischen Kontrollsubjektes, das nicht an SIRS oder Sepsis erkrankt ist, und einem Reagenz, welches auf Sauerstoffintermediate (ROI) anspricht und eine Farb-, Licht- oder eine andere meßbare Reaktion eingeht, in Kontakt bringt,
- die Farb-, Licht- oder anderen meßbaren Reaktionen in den Ansätzen mißt und die Meßwerte des zu untersuchenden Subjektes denjenigen des Kontrollsubjektes gegenüberstellt, wobei je schwerer das Krankheitsbild ist, desto niedriger ist die Menge der mit einer Patientenprobe gebildeten Sauerstoffintermediate (ROI) und damit auch die gemessene Reaktion.

Bei dem erfindungsgemäßen Verfahren werden anstelle nativer Leukozyten permanente Zellinien eingesetzt, die trotz uneingeschränkter Teilungsfähigkeit wesentliche Leukozytenfunktionen (Phagozytose, ROI-Produktion, Cytokin-, Chemokinausschüttung) beibehalten haben. Innerhalb der Population besitzen die erfindungsgemäß verwendeten Zellinien eine einheitliche und über viele Generationen konstante Reaktionsfähigkeit, weshalb ihr Einsatz als Sensorzellen im erfindungsgemäßen Testverfahren erheblich besser reproduzierbare und vergleichbare Ergebnisse liefert als beispielsweise native Leukozyten.

In einer erfindungsgemäß ganz besonders bevorzugten Ausführungsform ist die verwendete Zellinie daher eine leukozytäre Zellinie oder eine Leukozytenzellinie.

Wesentlich im Sinne der Erfindung ist, daß permanente Zellinien nach geeigneten Differenzierungsschritten durch die Applikation einer Probe, wie z. B. Humanserum, spontan zur Produktion von ROI angeregt werden. Zusätzlich auslösende Agentien wie Mikropartikel, N-fMLP oder PMA, wie bei Oxyburst-Messungen an nativen Leukozyten üblich, werden nicht eingesetzt.

Zellinien, die dabei zum Einsatz kommen, sind erfindungsgemäß solche, die auf Indikatoren für das Vorhandensein oder Fehlen von Indikatoren für SIRS oder Sepsis in einer Probe unter Freisetzung von Sauerstoffintermediaten (ROI) ansprechen, wobei die Menge der freigesetzten Sauerstoffintermediate (ROI) mit der Konzentration an Indikatoren für SIRS oder Sepsis in einer Probe korreliert.

Geeignete Zellinien umfassen zum Beispiel durch Hybridisierung von nativen Monozyten bzw. Makrophagen mit geeigneten Tumorzellen und anschließender Klonung etablierte Linien, wie sie in der US-A-4,737,455 beschrieben sind. Andere geeignete Zellinien lassen sich auf Isolate aus spontan entstandenen oder induzierten Tumoren (z. B. Leukämien) zurückführen, wobei einige noch durch zusätzliche Induktionsschritte zu phagozytierenden und ROI produzierenden Zellen ausdifferenziert werden müssen. Eine dominierende Rolle unter diesen Zelltypen fällt dabei der humanen promyelozytischen HL-60-Linie zu (Breitman TR et al. 1980, Collins SJ 1987). In einfachen Kulturmedien mit der stabil bleibenden Charakteristik von Vorläuferzellen unbegrenzt vermehrbar, können HL-60-Zellen durch spezifische Induktoren in granulozytäre (z. B. durch all-trans-Vitamin A-Säure) oder monozytäre Zellen (z. B. durch Vitamin D3-Derivate) ausdifferenziert werden. Nach Induktion der terminalen Ausdifferenzierung sind diese Zellen nur noch für begrenzte Zeit teilungsfähig und enden in einer homogenen Population, die in vielen Eigenschaften nativen Granulozyten bzw. Monozyten gleicht. Ihre leichte Handhabung und Beeinflußbarkeit prädestiniert die HL-60-Linie zur Nutzung als Sensorzellen im klinischen und pharmazeutischen Bereich. Erfindungsgemäß bevorzugte Zellinien sind HL-60 (ATCC, CCL-240), THP-1 (ATCC, TIP-202 bzw. DSMZ, ACC 16) und U937 (ATCC, CRL-1593.2 bzw. DSMZ, ACC 5).

Bisher angewandte Überprüfungen der Leukozytenfunktionen Phagozytose und Rot-produktion im Vollblut eines Patienten oder in Plasma nach Erythrozytensedimentation, die mit Durchflußzytometrie, Fluorometrie, Fluoreszenzmikroskopie und Chemilumineszenzmessung erfolgen, differenzieren bei Sepsis-Patienten nicht zwischen der gegebenen Funktionstüchtigkeit der Leukozyten und den immunmodulatorischen Einflüssen von Plasmafaktoren. Insbesondere bei Fluorometrie und Chemilumineszenzmessung entsteht eine zusätzliche Variabilität durch die schwankende jeweilige Leukozytenzahl. Der erfindungsgemäße Einsatz etablierter und hinsichtlich ihrer Eigenschaften gut untersuchter Zellinien bietet dagegen einen Test, der von den Schwankungen bezüglich der Funktionstüchtigkeit der Sensorzellen weitestgehend unabhängig ist.

Darüber hinaus besteht bei Verwendung von aufgereinigten Leukozyten in einem solchen Test die Gefahr der Voraktivierung noch ruhender Zellen während der Reinigungsprozedur, wodurch die Reproduzierbarkeit und Zuverlässigkeit der Messungen deutlich beeinträchtigt werden. Außerdem bedeutet das Arbeiten mit aufgereinigten Leukozyten für ein Routinelabor einen hohen methodischen und zeitlichen Aufwand, der durch das Verfahren der vorliegenden Erfindung und die Bereitstellung eines entsprechenden Testkits deutlich herabgesetzt werden kann.

In einem Aspekt ersetzt die vorliegende Erfindung daher bei der Bestimmung der Menge an Indikatoren für SIRS bzw. Sepsis in Körperflüssigkeiten, bevorzugterweise in Blut, Isolate von nativen Leukozyten durch reproduzierbar erhältliche und leicht standardisierbare Biosensoren.

Der Nachweis der gebildeten Sauerstoffintermediate (ROI) erfolgt über eine meßbare Reaktion, vorzugsweise eine Lichtreaktion. Ganz besonders bevorzugt ist der Nachweis über eine Chemilumineszenzreäktion. Besonders bevorzugte Reagenzien für die Chemilumineszenzreaktion sind Bis-N-Methylacridiniumnitrat (Lucigenin) und 5-Amino-1,2,3,4-tetrahydrophthalazin-1,2-dion (Luminol), die einzeln oder auch im Gemisch eingesetzt werden können. Ganz besonders bevorzugt ist das Reagenz Lucigenin.

Chemilumineszenz wird zweckmäßigerweise mittels eines Luminometers gemessen. Geeigneterweise führt man bei einer solchen Messung eine Kinetik über einen Zeitraum von vorzugsweise 2-120 min durch.

Überraschenderweise wurde festgestellt, daß die nach dem erfindungsgemäßen Verfahren ausgelöste Chemilumineszenz bei Serumproben aus einem Kollektiv von SIRS- bzw. Sepsis-Patienten Abweichungen von den Werten gesunder Probanden zeigte. Mit zunehmender Schwere des Krankheitsverlaufs sinkt die durch die Serumprobe verursachte Chemilumineszenz unter den Normbereich, den man bei gesunden Probanden ermittelt. Je schwerer das Krankheitsbild eines SIRS- oder Sepsispatienten ist, desto niedriger ist somit die Menge der in dem erfindungsgemäßen Verfahren mit einer Patientenprobe gebildeten Sauerstoffintermediate (ROI) und damit auch die gemessene Reaktion, z. B. Chemilumineszenz, mit dem eingesetzten Reagenz. Die schwere der Krankheit und das Letalitätsrisiko von SIRS- oder Sepsispatienten läßt sich durch das erfindungsgemäße Verfahren schon am Tag der Einweisung durch die erniedrigte Reaktion mit dem eingesetzten Reagenz, wie z. B. erniedrigte Chemilumineszenzwerte, erkennen. Erforderlich ist dabei in jedem Fall ein Vergleich mit den entsprechenden Werten einer oder mehrerer Kontrollproben von gesunden Patienten.

Durch einen relativ einfachen und billigen Test können so zeitaufwendige Scoring-Systeme oder aufwendige und teure Bestimmungen wie ELISAs und FACS-Analysen ersetzt werden. Basierend auf einer abgesicherten Korrelation mit SIRS/Sepsis-Verläufen kann das beschriebene Testverfahren ein schnell und leicht ermittelbare Indikation für therapeutische Maßnahmen liefern.

In einer weiteren bevorzugten Ausführungsform des erfindungsgemäßen Verfahrens ist die zum Nachweis der gebildeten Sauerstoffintermediate (ROI) eingesetzte meßbare Reaktion eine Fluoreszenzreaktion. Als Reagenzien hierfür eignen sich besonders Dihydrorhodamin und Hydroethidium oder ein Gemisch aus beidem. Die Messung der Fluoreszenzreaktion erfolgt in diesem Falle zweckmäßigerweise mittels Fluorometrie (intrazellulär und extrazellulär) oder Durchflußzytometrie (intrazellulär). Ein Vorteil davon ist, daß mittels Fluorometrie und Durchflußzytometrie auch intrazelluläre Fluoreszenzreaktion gemessen werden kann, die innerhalb der Sensorzellen mit in die Zellen eingedrungenen Reagenz stattfindet.

In einer weiteren bevorzugten Ausführungsform des erfindungsgemäßen Verfahrens ist die zum Nachweis der gebildeten Sauerstoffintermediate (ROI) eingesetzte meßbare Reaktion eine Farbreaktion. Als Reagenzien hierfür eignen sich besonders Eisen-III-Cytochrom, Nitrotetrazolium oder ein Benzidinderivat, vorzugsweise 3,3'-5,5'-Tetramethylbenzidin, oder ein Gemisch aus wenigstens zwei der vorgenannten. Die Messung der Farbreaktion erfolgt in diesem Falle zweckmäßigerweise spektralphotometrisch.

Erfindungsgemäß besonders zweckmäßig ist es , wenn die Zellen der Zellinie in Kultur vor dem Inkontaktbringen mit dem Blut, Blutplasma oder Blutserum, anderen Körperflüssigkeiten oder Lavagen oder Bestandteilen davon für einen Zeitraum mit Induktoren der Leukozytendifferenzierung, vorzugsweise mit Interferon gamma (IFN) und/oder 1-alpha-25-Dihydroxycholecalciferol (VD3) und/oder Granulozytenkolonie-stimulierendem Faktor (G-CSF) und/oder Tocoferol und/oder all-trans-Vitamin A-Säure, behandelt werden.

Die vorliegende Erfindung umfaßt neben dem erfindungsgemäßen Verfahren auch ein entsprechendes Test-Kit mit Zellen wenigstens einer permanenten, differenzierten leukozytären Zellinie, die ohne zusätzliche auslösende Agenzien auf Indikatoren für das Vorhandensein oder Fehlen von Indikatoren für SIRS bzw. Sepsis unter Freisetzung von Sauerstoffintermediaten (ROI) anspricht, Kontrollproben von Blut, Blutserum oder Blutplasma, anderen Körperflüssigkeiten oder Lavagen oder Bestandteilen davon eines Kontrollsubjektes ohne SIRS oder Sepsis und wenigstens einem Reagenz, welches auf Sauerstoffintermediate (ROI) anspricht und eine Farb-, Licht- oder eine andere meßbare Reaktion eingeht.

### Beispiele

Die Erfindung wird nun anhand von nicht beschränkenden Beispielen weiter erläutert. In den Beispielen wurden zu Vergleichszwecken verschiedene kommerziell erhältliche Zellinien für das erfindungsgemäße Testverfahren auf teilweise verschiedene Art vorbehandelt und anschließend in dem Verfahren mit Blutplasma von Sepsispatienten eingesetzt.

### Beispiel 1: Testsystem HL-60 CH04

HL-60-Zellen (ATCC, CCL-240) wurden nach Empfehlung des Herstellers herangezüchtet und anschließend in serumfreies Medium, z. B. Pro-CHO4-CDM (Biowhittaker, 12-029Q) mit 2 mM Glutamax (Invitrogen, 35050-038) übertragen. Die Zellen wurden alle 2-3 Tage mit einem kompletten Mediumwechsel und einer Anfangsdichte von 0,5×10E6 Zellen/ml passagiert und unter einer Atmosphäre mit 5 % CO₂ bei 37°C gehalten.

### Beispiel 2: Testsystem HL-60 VD3/IFN:

HL-60-Zellen (ATCC, CCL-240) wurden nach Empfehlung des Herstellers herangezüchtet und anschließend in DMEM (Invitrogen, 11880-028) mit 2 mM Glutamax (Invitrogen, 35050-038) und 10 % FBS (Invitrogen, 10099-141) wie oben beschrieben passagiert und unter einer Atmosphäre mit 5 % CO₂ bei 37 °C gehalten. Nach 5-tägiger Inkubation mit 1000 IU Interferon gamma (IFN) (Imukin, Boehringer Ingelheim) und 50 nM 1-alpha-25-Dihydroxycholecalciferol (VD3) (Biomol, DM200-1000) mit einem Mediumwechsel nach 3 Tagen wurden die adhärenten Zellen für den Testeinsatz geerntet.

### Beispiel 3: Testsystem THP-1 CHO4:

THP-1-Zellen (ATCC, TIP-202) wurden nach Empfehlung des Herstellers herangezüchtet und anschließend in serumfreies Medium, z. B. Pro-CHO4-CDM (Biowhittaker 12-029Q) mit 2 mM Glutamax (Life Technologies, 35050-038) übertragen. Die Zellen wurden alle 2-3 Tage mit einem kompletten Mediumwechsel und einer Anfangsdichte von 0,4x10E6 Zeilen/ml passagiert und unter einer Atmosphäre mit 5 % CO₂ bei 37 °C gehalten.

### Beispiel 4: Testsystem U-937 VD3/IFN:

U-937-Zellen (ATCC, CRL-1593.2) wurden nach Empfehlung des Herstellers herangezüchtet und anschließend in RPMI 1640 mit Glutamax (Invitrogen, 61870-010), 1 mM Natriumpyruvat (Invitrogen, 11360-039) und 10 % FBS (Invitrogen, 10099-141) kultiviert (5% CO₂ 37 °C, Anfangsdichte 0,3x10E6 Zellen/ml, kompletter Mediumwechsel nach 2-3 Tagen). Nach 3-tägiger Inkubation mit 1000 IU Interferon gamma (IFN) (Imukin, Boehringer Ingelheim) und 50 nM 1-alpha-25-Dihydroxycholecalciferol (VD3) (Biomol, DM200-1000) wurden die Zellen für den Testeinsatz geerntet.

### Beispiel 5: Testdurchführung

Die Zellen wurden jeweils am Ende der Passage aus dem Medium abzentrifugiert (250 xg, 5 min), anschließend in serumfreiem Medium, z. B. Pro-CHO4-CDM (Biowhittaker 12-0290) mit 2 mM Glutamax (Life Technologies, 35050-038) aufgenommen und auf eine Zelldichte von 10E6 Zellen/ml eingestellt. Die Zellen wurden für maximal 8 h bei Raumtemperatur aufbewahrt.

Jeweils 0,050 ml Humanserum wurden in den Näpfen einer weißen, nicht transparenten 96-Well-Mikrotiterplatte (z. B. Greiner, 655075) mit 0,050 ml 0,65 mM Lucigenin in PBS versetzt. Für die Nullpunktbestimmung ohne stimulierende Probe wurden statt Humanserum 0,050 ml PBS vorgelegt.

Nach Hinzupipettieren von jeweils 0,100 ml Zellsuspension (10E5 Zellen) wurde die Chemilumineszenzanregung jeder Probe mit Hilfe eines Luminometers (z. B. Luminoskan RS, Labsysterns) über einen Zeitraum von 45 Minuten verfolgt, wobei im Abstand von 3 min jeweils für 1 s das Integral der Meßsignale erfaßt wurde. Zur Auswertung wurde für jede Probe die Summe der Einzelintegrale prozentual zu den entsprechenden Werten der Kontrollen (Seren von gesunden Probanden) dargestellt.

Die Ergebnisse sind in den Figuren 1 bis 4 graphisch dargestellt. Sie zeigen jeweils den Vergleich der Reaktivität der Testsysteme HL-60 CHO4 (Figur 1), HL-60 VD3/IFN (Figur 2), THP-1 CHO4 (Figur 3) und U-937 VD3/IFN (Figur 4) bei mit septischem Schock eingewiesenen überlebenden Patienten (P01, P03) bzw. nicht überlebenden (NS) Patienten (P02, P04, P05). Der Wert von 100% entspricht dem Wert, der mit Serum von gesunden Probanden erzielt wurde.

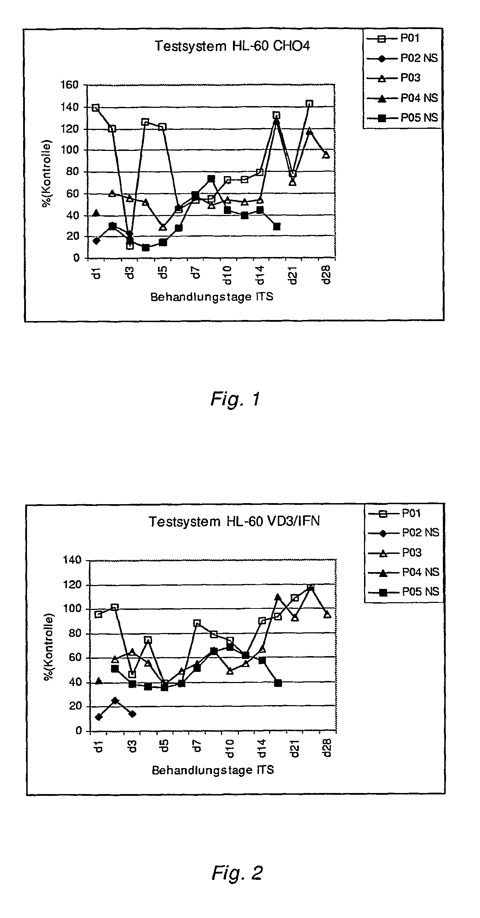

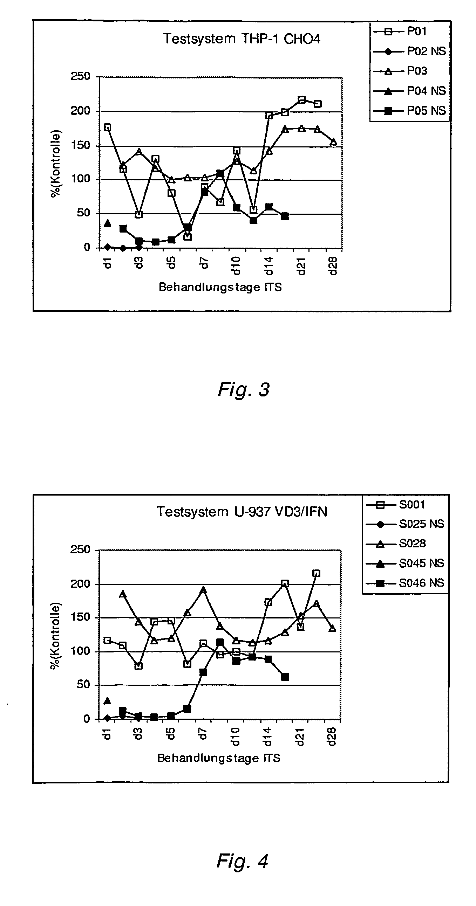

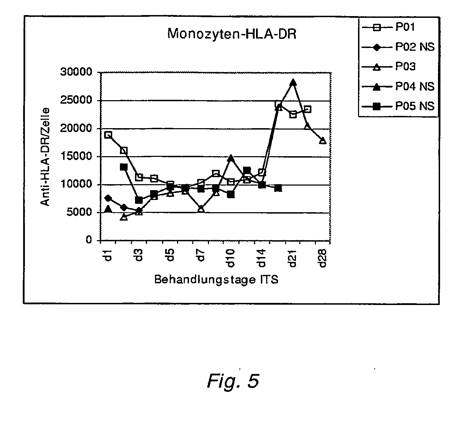

Zum Vergleich ist in Figur 5 der Verlauf der Immunsuppression anhand der HLA-DR-Expression auf Monozyten dargestellt, welche mittels eines handelsüblich erhältlichen Tests gemessen wurde (Testkit Quantibrite Anti-HLA-DR, Becton-Dickinson 340827). In diesem Vergleichsexperiment wurden die gleichen Patientenproben eingesetzt wie in den Tests gemäß den Figuren 1 bis 4. Ein Vergleich der Ergebnisse in den Figuren 1 bis 4 mit denjenigen der Figur 5 zeigt deutlich, daß das erfindungsgemäße Verfahren wesentlich besser differenziert zwischen dem Krankheitsbild von Patienten mit hoher Überlebenserwartung (P01, P03) und solchen mit einem demgegenüber hohen Letalitätsrisiko (P02, P04, P05).

Bezug nehmend auf die in den Figuren 1 bis 4 dargestellten Ergebnisse, zeigt der Anstieg der gemessenen Chemilumineszenzsignale bei den überlebenden Patienten P01 und P03 während der Behandlung deutlich die einsetzende Genesung, wogegen die Patienten P02, P04 und P05 entweder erheblich früher (P02 und P04) oder etwa am Tag 18 der Behandlung (P05) verstarben. Sehr deutlich ist die überraschende Korrelation der Stärke der Chemilumineszenz am Tag der Einlieferung und kurz danach mit der Tatsache, ob die Patienten durch die Behandlung genesen konnten oder trotz der Behandlung verstarben (Korrelation zwischen Letalität und anfänglichen Chemilumineszenzsignalen). Eine relativ hohe Chemilumineszenz am Tag der Einlieferung und kurz darauf deutete auf eine wesentlich höhere Überlebenswahrscheinlichkeit hin als eine relativ niedrige Chemilumineszenz in dieser Zeit. Die hohe Chemilumuszenzenz am Tag der Einlieferung und kurz danach bei den Überlebenden Patienten, die teilweise sogar deutlich über den Meßwerten bei gesunden Kontrollpatienten lag, deutet auf eine starke Stimulation und Anregung des Immunsystems dieser Patienten hin. Solche Patienten haben daher eine gute Überlebensprognose. Bei den Patienten, die später verstarben, war nur eine erheblich geringere Chemiluminszenzreaktion festzustellen, was auf ein bereits am Tag der Einlieferung mehr oder weniger stark geschwächtes Immunsystem dieser Patienten hindeutet.

Diese Tatsache war einerseits überraschend und eröffnet andererseits aufgrund einer sehr frühen Diagnostizierbarkeit des Letalitätsrisikos eines Patienten die Möglichkeit, weitergehende therapeutische Maßnamen zu ergreifen als die bei solchen Krankheitsbildern üblichen. Darüber hinaus bietet der erfindungsgemäße Test ein sehr einfaches, vergleichsweise preiswertes und vor allem schnelles und zuverlässiges Mittel, das Letalitätsrisiko von SIRS- und Sepsispatienten einzustufen. Die Reproduzierbarkeit und Vergleichbarkeit des Tests wird durch die erfindungsgemäße Verwendung von standardisierten und reproduzierbaren Zellsystemen als Biosensoren erst möglich.

### Literatur

Grimminger F et al.: Internist 38, 541-552 (1997)
Romaschin AD et al.: Sepsis 2, 119-125 (1998)
Oberhoffer M et al.: Clin Chem Lab Med 37(3), 363-368 (1999)
Mimoz et al.: Intensive Care Med 24, 185-188 (1998)
Al-Nawas B et al.: Eur J Med Res 1, 331-333 (1995/96)
Nussler AK et al.: Langenbeck's Arch Surg 384, 222-232 (1999)
Wakefield CH et al.: Brit J Surg 80, 205-209 (1993)
Asadullah K et al.: Crit Care Med 23,1976-1983 (1995)
Döcke WD et al.: Nature Med 3, 678-681 (1997)
Pascual et al.: Intensive Care med 23, 743--748 (1997)
Breitman TR et al.: Proc Natl Acad Sci USA 77(5), 2936-2940 (1980)
Collins SJ: Blood 70(5), 1233-1244 (1987)
Dong YL et al.: J Trauma 34(3), 417-421 (1993)
Holzer K et al.: 5th World Congress on Trauma, Shock, Inflammation and Sepsis, Faist E ed., Monduzzi Editori publ., 593-597 (2000)
Bone RC et al.: Chest 101(6), 1644-1654
Knaus WA et al.: Crit Care Med 13(10), 818-829

## Patentansprüche

1. Verfahren zur extrakorporalen qualitativen oder semiquantiativen Bestimmung der Menge an Indikatoren für Systemic Inflammatory Response System (SIRS) bzw. Sepsis im Blut, Blutserum, Blutplasma, anderen Körperflüssigkeiten oder Lavagen oder Bestandteilen davon von menschlichen oder tierischen Subjekten, wobei das Verfahren die Stufen umfaßt, in denen man
- eine permanente, differenzierte leukozytäre Zellinie in Kultur bereitstellt, die ohne zusätzliche auslösende Agenzien auf Indikatoren für das Vorhandensein oder Fehlen von Indikatoren für SIRS bzw. Sepsis unter Freisetzung von Sauerstoffintermediaten (ROI) anspricht,
- in wenigstens einem ersten Ansatz Zellen der Zellinie mit Blut, Blutserum oder Blutplasma, anderen Körperflüssigkeiten oder Lavagen oder Bestandteilen davon eines zu untersuchenden menschlichen oder tierischen Subjektes und einem Reagenz, welches auf Sauerstoffintermediate (ROI) anspricht und eine Farb-, Licht- oder eine andere meßbare Reaktion eingeht, in Kontakt bringt,
- in wenigstens einem weiteren Ansatz Zellen der Zellinie mit Blut, Blutserum oder Blutplasma, anderen Körperflüssigkeiten oder Lavagen oder Bestandteilen davon eines menschlichen oder tierischen Kontrollsubjektes, das nicht an SIRS oder Sepsis erkrankt ist, und einem Reagenz, welches auf Sauerstoffintermediate (ROI) anspricht und eine Farb-, Licht- oder eine andere meßbare Reaktion eingeht, in Kontakt bringt,
- die Farb-, Licht- oder anderen meßbaren Reaktionen in den Ansätzen mißt und die Meßwerte des zu untersuchenden Subjektes denjenigen des Kontrollsubjektes gegenüberstellt, wobei je schwerer das Krankheitsbild ist, desto niedriger ist die Menge der mit einer Patientenprobe gebildeten Sauerstoffintermediate (ROI) und damit auch die gemessene Reaktion.

2. Verfahren nach Anspruch 1, **dadurch gekennzeichnet, daß** die meßbare Reaktion eine Lichtreaktion, vorzugsweise eine Chemilumineszenzreaktion ist.

3. Verfahren nach einem der Anspüche 1 oder 2, **dadurch gekennzeichnet, daß** das Reagenz Bis-N-Methylacridiniumnitrat (Lucigenin), 5-Amino-1,2,3,4-tetrahydrophthalazin-1,2-dion (Luminol) oder Gemische davon, vorzugsweise Lucigenin ist.

4. Verfahren nach einem der Ansprüche 2 oder 3, **dadurch gekennzeichnet, daß** die Chemilumineszenz mittels eines Luminometers, vorzugsweise durch eine Kinetik über einen Zeitraum von 2-120 min, gemessen wird.

5. Verfahren nach Anspruch 1, **dadurch gekennzeichnet, daß** die meßbare Reaktion eine Fluoreszenzreaktion ist.

6. Verfahren nach einem der Anspüche 1 oder 5, **dadurch gekennzeichnet, daß** das Reagenz Dihydrorhodamin, Hydroethidium oder ein Gemisch aus beidem ist.

7. Verfahren nach einem der Anspüche 1, 5 oder 6, **dadurch gekennzeichnet, daß** die Fluoreszenzreaktion mittels Fluorometrie oder Durchflußzytometrie gemessen wird.

8. Verfahren nach Anspruch 1, **dadurch gekennzeichnet, daß** die meßbare Reaktion eine Farbreaktion ist.

9. Verfahren nach einem der Anspüche 1 oder 8, **dadurch gekennzeichnet, daß** das Reagenz Eisen-III-Cytochrom, Nitrotetrazolium oder ein Benzidinderivat, vorzugsweise 3,3'-5,5'-Tetramethylbenzidin, oder ein Gemisch aus wenigstens zwei der vorgenannten ist.

10. Verfahren nach einem der Anspüche 1, 8 oder 9, **dadurch gekennzeichnet, daß** die Farbreaktion spektralphotometrisch gemessen wird.

11. Verfahren nach einem der vorangegangenen Ansprüche, **dadurch gekennzeichnet, daß** die Zellen der Zellinie HL-60-Zellen (ATCC, CL-240), THP-1-Zellen (ATCC, TIP-202) oder U-937-Zellen (ATCC, CRL-1593.2) sind.

12. Verfahren nach einem der vorangegangenen Ansprüche, **dadurch gekennzeichnet, daß** die Zellen der Zellinie in Kultur vor dem Inkontaktbringen mit dem Blut, Blutplasma oder Blutserum, anderen Körperflüssigkeiten oder Lavagen oder Bestandteilen davon für einen Zeitraum mit Induktoren der Leukozytendifferenzierung, vorzugsweise mit Interferon gamma (IFN) und/oder 1-alpha-25-Dihydroxycholecalciferol (VD3) und/oder Granulozytenkolonie-stimulierendem Faktor (G-CSF) und/oder Tocoferol und/oder all-trans-Vitamin A-Säure, behandelt werden.

13. Test-Kit mit Zellen wenigstens einer permanenten, differenzierten leukozytären Zellinie, die ohne zusätzliche auslösende Agenzien auf Indikatoren für das Vorhandensein oder Fehlen von Indikatoren für SIRS bzw. Sepsis unter Freisetzung von Sauerstoffintermediaten (ROI) anspricht, Kontrollproben von Blut, Blutserum oder Blutplasma, anderen Körperflüssigkeiten oder Lavagen oder Bestandteilen davon eines Kontrollsubjektes ohne SIRS oder Sepsis und wenigstens einem Reagenz, welches auf Sauerstoffintermediate (ROI) anspricht und eine Farb-, Licht- oder eine andere meßbare Reaktion eingeht.

## Claims

1. Method for the extracorporeal qualitative or semi-quantitative determination of the quantity of indicators for Systemic Inflammatory Response System (SIRS) or sepsis in the blood, blood serum, blood plasma, other body fluids or lavages or constituents thereof of human or animal subjects, wherein the method comprises the steps in which
- a permanent, differentiated leukocytary cell line culture is provided which reacts by releasing oxygen intermediates (ROI) without additional triggering agents to indicators for the presence or absence of indicators for SIRS or sepsis, respectively,
- in at least one first preparation, cells of the cell line are brought into contact with blood, blood serum or blood plasma, other body fluids or lavages or constituents thereof of a human or animal subject to be examined, and with a reagent which responds to oxygen intermediates (ROI) and enters into a colour, light or other measurable reaction,
- in at least one further preparation, cells of the cell line are brought into contact with blood, blood serum or blood plasma, other body fluids or lavages or constituents thereof of a human or animal control subject not ill with SIRS or sepsis, and with a reagent which responds to oxygen intermediates (ROI) and enters into a colour, light or other measurable reaction,
- the colour, light or other measurable reactions are measured in the preparations and the measured values of the subject to be examined are compared with those of the control subject, whereby the more severe the disease pattern is, the lower the amount of oxygen intermediates (ROI) produced in the patient's sample and thus the measured reaction is.

2. Method according to claim 1, **characterized in that** the measurable reaction is a light reaction, preferably a chemiluminescence reaction.

3. Method according to one of claims 1 or 2, **characterized in that** the reagent is bis-N-methylacridiniumnitrate (lucigenin), 5-amino-1,2,3,4-tetrahydro-phthalazine-1,2-dione (luminol) or mixtures thereof, preferably lucigenin.

4. Method according to one of claims 2 or 3, **characterized in that** the chemiluminescence is measured by means of a luminometer, preferably by a kinesis over a period of 2-120 min.

5. Method according to claim 1, **characterized in that** the measurable reaction is a fluorescence reaction.

6. Method according to one of claims 1 or 5 **characterized in that** the reagent is dihydrorhodamine, hydroethidium or a mixture of the two.

7. Method according to one of claims 1, 5 or 6, **characterized in that** the fluorescence is measured by means of fluorometry or throughflow cytometry.

8. Method according to claim 1, **characterized in that** the measurable reaction is a colour reaction.

9. Method according to one of claims 1 or 8, **characterized in that** the reagent is iron-III-cytochrome, nitrotetrazolium or a benzidine derivative, preferably 3,3-5,5-tetramethylbenzidine, or a mixture of at least two of the aforementioned.

10. Method according to one of claims 1, 8 or 9, **characterized in that** the colour reaction is measured by means of spectral photometer.

11. Method according to one of the previous claims, **characterized in that** the cells of the cell line are HL-60 cells (ATCC, CCL-240), THP-1 cells (ATCC, TIP-202) or U-937 cells (ATCC, CRL-1593.2).

12. Method according to one of the previous claims, **characterized in that**, before being brought into contact with the blood, blood plasma or blood serum, other body fluids or lavages or constituents thereof, the cells of the cell line are treated in culture for a period of time with inductors of the leukocyte differentiation, preferably with interferon gamma (IFN) and/or 1-alpha-25-dihydroxycholecalciferol (VD3) and/or granulocyte colony- stimulating factor (G-CSF) and/or tocoferol and/or all-trans-vitamin A acid.

13. Test kit with cells of at least one permanent, differentiated leukocytary cell line which reacts by releasing oxygen intermediates (ROI) without additional triggering agents to indicators for the presence or absence of indicators for SIRS or sepsis, respectively, control samples of blood, blood serum or blood plasma, other body fluids or lavages or constituents thereof of a control subject without SIRS or sepsis and at least one reagent which responds to oxygen intermediates (ROI) and enters into a colour, light or other measurable reaction.

## Revendications

1. Procédé de détermination extracorporelle qualitative ou semi-quantitative de la quantité d'indicateurs du syndrome de réponse inflammatoire systémique (SIRS) ou de la septicémie présente dans le sang, le sérum sanguin, le plasma sanguin, d'autres liquides corporels ou liquides de lavage ou des constituants desdits liquides provenant de sujets humains ou d'animaux, ce procédé comportant les étapes consistant à :
- préparer une lignée cellulaire leucocytaire différenciée et permanente mise en culture qui sans agents déclencheurs supplémentaires réagit sur les indicateurs pour la présence ou l'absence d'indicateurs du SIRS ou de la septicémie en libérant des produits intermédiaires d'oxygène réactif (ROI),
- mettre en contact, dans au moins une première préparation, des cellules de la lignée cellulaire avec du sang, du sérum sanguin ou du plasma sanguin, d'autres liquides corporels ou liquides de lavage ou des constituants desdits liquides provenant d'un sujet humain ou d'un animal à examiner avec un réactif, lequel réagit à des produits intermédiaires d'oxygène réactif (ROI) et entre dans une réaction colorée, une photo-réaction ou toute autre réaction mesurable,
- mettre en contact, dans au moins une autre préparation, des cellules de la lignée cellulaire avec du sang, du sérum sanguin ou du plasma sanguin, d'autres liquides corporels ou liquides de lavage ou des constituants desdits liquides provenant d'un sujet humain témoin ou d'un sujet animal témoin n'étant pas atteint du SIRS ou de la septicémie avec un réactif, lequel réagit à des produits intermédiaires d'oxygène réactif (ROI) et entre dans une réaction colorée, une photo-réaction ou toute autre réaction mesurable,
- mesurer les réactions colorées, les photo-réactions ou d'autres réactions mesurables dans les préparations et à comparer les valeurs de mesure du sujet à examiner à celles du sujet témoin, au moyen duquel, plus le schéma pathologique est élevé, plus la quantité de produits intermédiaires d'oxygène réactif (ROI) composée d'un échantillon prélevé chez un patient est faible et par conséquent la réaction mesurée également.

2. Procédé selon la revendication 1 **caractérisé en ce que** la réaction mesurable est une photo-réaction, de préférence une réaction chimioluminescente.

3. Procédé selon l'une des revendications 1 ou 2 **caractérisé en ce que** le réactif Bis-N-méthylacridinium nitrate (lucigénine) est du 5-amino-1, 2, 3,4-tétrahydrophthalazine-1,2-dione (luminol) ou des mélanges de ceux-ci et est de préférence de la lucigénine.

4. Procédé selon l'une des revendications 2 ou 3 **caractérisé en ce que** la chimioluminescence est mesurée par un luminomètre, de préférence par une cinétique sur une période de temps de 2 à 120 minutes.

5. Procédé selon la revendication 1 **caractérisé en ce que** la réaction mesurable est une réaction fluorescente.

6. Procédé selon l'une des revendications 1 ou 5 **caractérisé en ce que** le réactif est de la dihydrorhodamine, du hydroéthidium ou un mélange des deux.

7. Procédé selon l'une des revendications 1, 5 ou 6 **caractérisé en ce que** la réaction fluorescente est mesurée par le biais d'une fluorométrie ou par cytométrie de flux.

8. Procédé selon la revendication 1, **caractérisé en ce que** la réaction mesurable est une réaction colorée.

9. Procédé selon l'une des revendications 1 ou 8, **caractérisé en ce que** le réactif est du fer-111-cytochrome, du nitrotétrazolium ou un dérivé de la benzidine, de préférence de la 3,3'-5,5'-tétraméthylbenzidine ou un mélange d'au moins deux des éléments susmentionnés.

10. Procédé selon l'un des revendications 1, 8 ou 9 **caractérisé en ce que** la réaction colorée est mesurée par spectrophotométrie.

11. Procédé selon l'une des revendications précédentes **caractérisé en ce que** les cellules de la lignée cellulaire sont des cellules HL-60 (ATCC, CL-240), des cellules THP-1 (ATCC, TIB-202) ou des cellules U-937 (ATCC, CRL-1593.2).

12. Procédé selon l'une des revendications précédentes **caractérisé en ce qu'**avant la mise en contact avec le sang, le plasma sanguin ou le sérum sanguin, d'autres liquides corporels ou liquides de lavage ou des constituants desdits liquides, les cellules de lignée cellulaire mise en culture sont traitées pendant une période avec des inducteurs de différenciation des leucocytes, de préférence avec l'interféron gamma (IFN) et/ou 1-alpha-25-dihydroxycholécalciférol (VD3) et/ou le facteur stimulant les colonies de granulocytes (G-CSF) et/ou le tocophérol et/ou l'acide all-trans-vitamine A.

13. Kit d'essai avec des cellules dont au moins une lignée cellulaire leucocytaire différenciée et permanente qui sans agents déclencheurs supplémentaires réagit sur les indicateurs pour la présence ou l'absence d'indicateurs du SIRS ou de la septicémie en libérant des produits intermédiaires d'oxygène réactif (ROI), échantillons de contrôle de sang, de sérum sanguin, de plasma sanguin, d'autres liquides corporels ou liquides de lavage ou des constituants desdits liquides d'un sujet témoin n'étant pas atteint du SIRS ou d'une septicémie et au moins un réactif, lequel réagit à des produits intermédiaires d'oxygène réactif (ROI) et entre dans une réaction colorée, une photo-réaction ou toute autre réaction mesurable.
